# EUROPEAN PATENT APPLICATION

(11) **EP 3 900 748 A1**
(43) Date of publication of application: **27.10.2021**
(21) Application number: 19898756.2
(22) Date of filing: 09.01.2019
(51) Int. Cl.: A61L 9/20, B01D 53/90

(54) **DEVICE FOR CLEANING AND STERILIZING AIR AND OBJECT SURFACE**

(30) Priority: 21.12.2018 CN 201811569356
(71) Applicant: Du, Guodong, Guangdong 518000 (CN)
(72) Inventor: Du, Guodong, Guangdong 518000 (CN)
(74) Representative: Mora Granell, José Agustín
(86) International application number: PCT/CN2019/070960
(87) International publication number: WO 2020/124706

(57) **Abstract**

A device for cleaning and sterilizing air and object surfaces, comprising an air control part for an air inlet that sucks air from an exterior connected to a propeller fan and conducts it to a main air channel with an ozone generator and a Peltier structures communicating with the main air channel. A Venturi channel introduces the gas in a mixing area with TiO2 and illuminated by a UV/LED lamp. In the mixing area, through reactions between the UV/LED lamp and the TiO2 before being discharged through a generation box 2. The generation box 2 may contain hydrogen peroxide and low-electric-conductivity deionized water without carbonate or bicarbonate.

## Description

### Technical field

The disclosure relates to a device for cleaning and sterilizing indoor air by an ozone, hydrogen peroxide, hydroxyl radical and radon removal function.

### Background

In a limited interior space under its little exchange with outdoor space and an indoor environment lacking natural purification ability, pollutants such as volatile organic compounds, solid suspended particulars, and aerosol with main pollutant components of formaldehyde and benzene are continuously discharged into the indoor space due to indoor decoration, popularization of air conditioners and daily life of people.

In some places of our country, the ground constitutive structure contains a large quantity of radon. Since radon is inert gas that does not react with other surrounding substances, certain 222Rn atoms will leave their birth places to be dispersed to other places through gaps between atoms or rock crystalline grains. 222Rn still has its α radioactive property and a half-life of 3.82 days, so it has enough time to be dispersed to a far place before decay, and even part of it is released from stone and dispersed to surrounding air, so radioactive decay will proceed in the air.

The presence of these pollutants not only makes people feel bad, but also affects people's health. At present, for indoor air purification and disinfection methods, people mainly adopt a pure mechanical adsorption type, an electrostatic type, a chemical (or physical) adsorption type, an anion type and a photocatalysis type, etc., since the pure mechanical adsorption type and the electrostatic dust removal type have a limited purifying effect and are mainly used for removing suspended particulars without a high purifying degree for organic pollution, they have gradually become obsolete. Products by other several techniques also each have advantages and disadvantages adapting to different pollutants and satisfying different customers. For instance, an adsorption purifier can adsorb specific or even most of organic pollutants according to characteristics of a selected adsorption material, but it has a saturation problem of its adsorption material. So there is a need to material regeneration and replacement. While anions are beneficial to decomposition of organic matter and converts the organic matter into basically harmless carbon dioxide, water and other inorganic matter, their products in a reaction process also have an adverse effect on human body to a certain extent and their ability of control over bacteria, viruses and chemical pollutants is also quite limited. Moreover, since the air purifier has difficulty in removing radon in the air at the same time, it is necessary to continuously develop new products with more functions, better effects, lower production cost and higher use convenience according to the increasing living standard to meet people's needs.

### Summary

The basis of any bactericidal principle lies in oxidizing an essential part of microorganisms to make unable to survive. Hydroxyl radicals are the most oxidizing substances in nature but harmless to human body. Hydroxyl radicals can stick to dust particles, causing them to decompose and eliminate the microorganisms contained within under the quite safe circumstance. Especially, hydroxyl radicals are also beneficial to precipitation of these particles and decomposed substances in humid environments.

The device for cleaning and sterilizing air and object surface of the disclosure sucks in exteradonal air through an air blower and other units, allows the exteradonal air to enter a Peltier condensation structure through a main air channel, and removes the adsorbed radon by condensing vapor in the air. Then, the air leaving the condensation structure comes through the main air channel again and enters a hydroxyl radical generation structure, and finally a large number of hydroxyl radicals are discharged into the exteradonal air for cleaning and sterilizing the air.

In the device for cleaning and sterilizing air and object surface of the disclosure, the hydroxyl radical generation structure communicates with an ozone generator, a generation box 1 and a generation box 2 through the main air channel, with corresponding circuit equipment disposed for supplying power to the ozone generator and piezoelectric membranes on the generation boxes and atomization units. Meanwhile, an ultraviolet lamp and a titanium dioxide coating are further disposed on the channel to form a titanium dioxide physical environment for physical-chemical reactions of mixed gas in the air channel, and thus hydroxyl radicals are acquired multiple times and are ultimately discharged into an outer space through the cover of the generation box 2 and the atomization unit thereon.

The device for cleaning and sterilizing air and object surface of the disclosure is to clean and sterilize air based on a combination of multiple technologies.

The hydroxyl radical generation structure of the disclosure comprises a photocatalysis unit with the UV/LED lamp, an ultrasonic decomposition unit with the generation boxes, and the ozone generator for generating ozone. The ozone generator comprises a boosted circuit, and continuously transfers ozone generated by the ozone generator to the air channel through the fan/air blower. The piezoelectric membranes in the generation boxes are configured to atomize liquid in the generation boxes and promote its micronization.

In addition, the ozone generated by the ozone generator and H₂O₂ in the generation boxes are mixed in a mixing channel and react to generate hydroxyl radicals. Deionized water and hydrogen peroxide are contained in the generation box 1, and finally H₂O, H₂O₂ and O₂ are generated. The gas in the generation box 1 is dragged to the mixing channel through a Venturi channel at the upper portion of the generation box 1 on the basis of a Venturi effect to further react with the ozone and other gas in the mixing channel, thereby further increasing the number of hydroxyl radicals.

H₂O, H₂O₂ and O₂ in the mixing channel chemically react with TiO₂ sprayed on the channel. Meanwhile, the gas in the channel undergoes ultraviolet radiation from ultraviolet light generated by the LED lamp and meanwhile continues to react with the mixed gas of H₂O, H₂O₂ and O₂ in the generation box 1 and is carried to the mixing channel by the Venturi effect, thereby further increasing the number of hydroxyl radicals.

Free radicals and hydroxyl radicals are discharged to an exteradonal closed space after being atomized by the generation box 2, thereby eliminating and precipitating pathogens in the air of the closed space and on surface of objects in the environment.

The hydroxyl radical generation unit of the disclosure comprises an air control part for an air inlet that sucks air from an exterior connected to a propeller fan. The main air channel contains ozone and forms the Venturi effect, and vapor, hydrogen peroxide and oxygen in the generation box 1 are driven by the main air channel to enter the main mixing channel (or referred to as a reaction chamber) so as to have a Venturi reaction by the UV/LED lamp in a pipeline coated with titanium dioxide.

The air control part is located between an air outlet and the air inlet and is configured to allow air flow to have effective physical-chemical reactions before the air flow makes contact with the atomization unit of the generation box 2.

The device further comprises one or more electronic circuits configured to control operation of various functions needing electricity, such as an ultrasonic wave generation unit and power supplies of the fan and the ozone generator.

The device for purifying and sterilizing air with an radon removal function of the disclosure comprises the condensation structure with a Peltier. A large quantity of radon is adsorbed to water drops condensed after the air enters the Peltier condensation structure through the main air channel to be condensed, and these water drops are stored in airtight porous sponge.

The disclosure attempts to remove the harmful substance radon, dissolved in vapor, in the air and generate a large number of hydroxyl radicals by a combination of the Peltier condensation structure and the hydroxyl radical generation structure, thereby comprehensively purifying the air and creating warm air flow near the device. The device can be used for cleaning and sterilizing air and object surface in closed spaces such as home, hotel, office building, hospital operating room, ward, train carriage, ocean-going vessel, military submarine, warship, aircraft carrier, war preparation pit, field hospital cabin and car.

### Brief Description of Figures

Fig. 1 is a side view of a generation box 1.
Fig. 2 is a side view of a generation box 2.
Fig. 3 is a schematic diagram of the device of the disclosure.

### Detailed Description

The disclosure attempts to radiate substances contained in the two generation boxes containing hydrogen peroxide and deionized water with ultraviolet light and makes them react with a substance that may generate hydroxyl radicals for physical-chemical chain reactions.

Low electric conduction and ultrasonic dissolution have three functions (decomposing water molecules, generating chitin resonance and fining water particles to generate cold vapor). When air flow passes through the generation boxes and filter elements, it can promote mixing and extracting of their contained substances and generate the Venturi effect to carry the contained substances to pass through a titanium dioxide channel. In such process, hydroxyl radicals, water, oxygen, hydrogen peroxide and ozone of the contained substances are separated and discharged into the air for sterilizing and cleaning the surrounding environment.

Therein, chitin starts to resonate with pillbug of chitin exoskeleton due to its resonance frequency, so it is particularly suitable for killing the pillbug.

The disclosure is further described in conjunction with accompanying drawings and embodiments as follows.

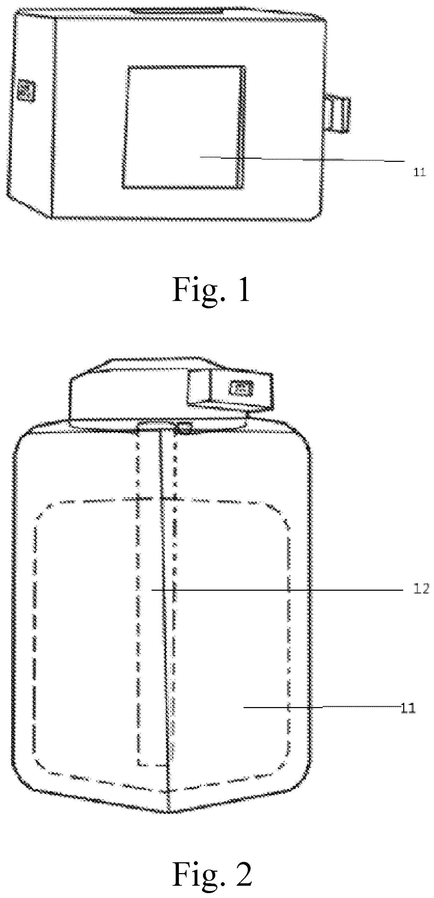

By reference to Fig.1, a device for cleaning and purifying air and body surface comprises an outer shell, and further comprises: 1, a fan/air blower; 2, an ozone generation unit and circuit; 3, a generation box 1; 4, a mixing channel; 5, a generation box 2; 6, a cover; 7, a Venturi channel; 8, a main air channel; 9, a UV/LED lamp; 10, a Peltier condensation structure; 11, liquid; and 12, a suction core.

Chemical reactions generated in the device are shown in Table 1.

The device comprises a combination of a hydroxyl radical generation structure and the Peltier condensation structure therein. The device comprises the air control part 1 for an air inlet that sucks air from an exterior connected to the propeller fan.

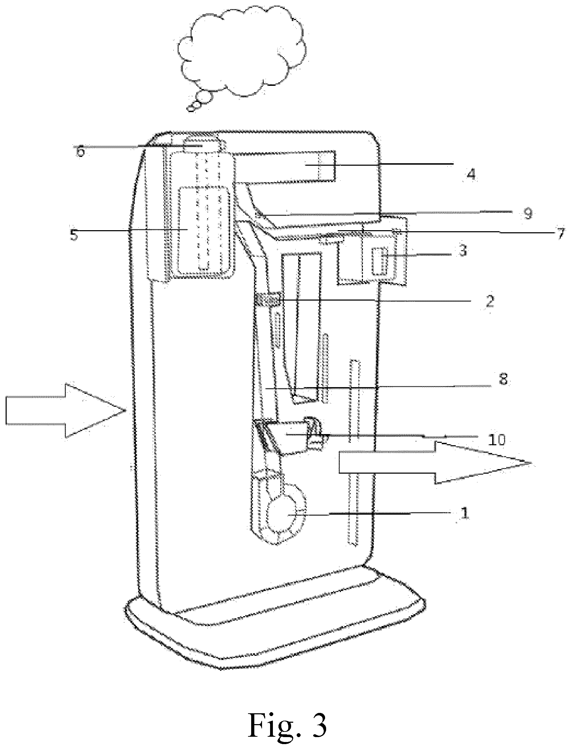

As shown in Fig. 3, air enters the main air channel 8 through the air control part and firstly passes through the Peltier condensation structure 10.

The Peltier condensation structure comprises a Peltier in the middle and also comprises a supply circuit. When a current flows through the Peltier, the two sides of the Peltier dissipate heat and absorb heat. As shown in Fig. 2, due to the heat absorption of the left side of the Peltier, vapor in the air flowing near the left side of the Peltier is condensed. Meanwhile, the condensation structure comprises a collecting unit, such as airtight porous sponge, configured to collect condensed water drops,

Due to the heat dissipation of the right side of the Peltier, it generates a large quantity of heat to provide stream of hot air flow, which is supplied out of the device by another fan. Thus, the temperature of surrounding air of the device will be raised.

The device comprises the two generation boxes, namely the generation box 1 and the generation box 2, configured to contain a solution, preferably peroxide water (hydrogen peroxide) and low-electric-conductivity deionized water with no carbonate or bicarbonate contained.

Ozone is generated by the ozone generation unit and circuit 2 with built-in ceramic electrodes and a built-in boosted circuit. The volume of ozone generated in the channel may be controlled by adjusting an activation time of the boosted circuit and a diameter of nozzles fixed to the electrodes (electrode areas), and thus ozone is captured to confirm to a volume of optimum standard demand. The ozone is driven by the fan 1 to pass through a central channel after being generated. Part of gas may be sucked into the generation box 1 based on the generated Venturi effect of the device. The generation box 1 contains hydrogen peroxide, vapor and oxygen. Therefore, the ozone is mixed with and reacts with these contained substances to generate gas of oxygen and ozone free radicals O°, H° and OH°.

Generated gas in the generation box 1 together with air and ozone in the main air channel enters the mixing channel 4 coated with titanium oxide, and at the moment, the UV/LED lamp 9 at the upper portion of the main air channel illuminates the mixing channel to activate more hydroxyl radicals. The gas of the main channel area is sent into the generation box 2 by the Venturi effect and discharged into exteradonal air by the top ultrasonic decomposition atomization unit of the generation box 2 together with the gas in the generation box 2.

The generation box 2 also contains liquid contained substances such as deionized water and hydrogen peroxide, and O² generated therein sufficiently reacts with ozone and hydrogen peroxide in a specific space through Venturi reaction air flow. Meanwhile, an ultrasonic generation piezoelectric sensor (converting electric energy into mechanical energy) with an atomizing function is added into the generation box 2 and disposed on its top, which is coated with TiO₂. It is known that the final mixed gas in the generation box 2 also generates more hydroxyl radicals.

The generation box 2 comprises a piezoelectric membrane. When ultrasonic waves of the piezoelectric membrane are conducted to the generation box 2, the contained liquid is absorbed by the ceramic core and decomposed ultrasonically through physical-chemical reactions for ultrasonic decomposition so as to be ionized into hydroxyl radicals OH°/H°O in a high oxide environment.

An ultrasonic wave generation part of the piezoelectric membrane of the generation box 2 supplies ultrasonic waves by a shell body at the upper portion of the generation box 2 for vibration for ultrasonic decomposition. An electrical part is further disposed and comprises a supply circuit and an oscillator providing another ultrasonic decomposition frequency, consistent with a chitin frequency and thus generates resonance to easily eliminate pillbug.

The generation box 2 is further equipped with the suction core 12 that is a suction part configured to capture the liquid in the generation box 2 and pump the liquid onto the piezoelectric membrane by a capillary effect. The core, preferably cellulose, extracts the contained liquid.

The piezoelectric membrane has a frequency of 15 Khz to 10 Mhz and has three effects of (i) ultrasonic decomposition, (ii) resonance with chitinous substances for weakening frameworks of mildew and yeast and eliminating aforementioned harmful substances, and (iii) atomization of the liquid from the generation box 2.

A UV/LED lamp 9 is further disposed on the top of the generation box 2.

The generation box 1 may be equipped with a suction core and a piezoelectric membrane like the generation box 2 or may be coated with TiO₂ and illuminated by the UV/LED lamp 9 in the same way. Therefore, the two generation boxes individually have a gas generation condition of H₂O₂+H₂O+O₃+O₂.

Such free radicals are added to other free radicals such as OH° or hydroxyl radicals and groups generated by an ozone decomposition effect to create reactive reactant conditions. In conjunction with air flowing and ultrasonic radiating of less than 390 nm, when vapor, oxide water, ozone and oxygen pass through the mixing channel 4 coated with TiO₂ to be dispersed in the air, their generated hydroxyl radicals are mixed with various ions as much as possible in the air so as to disperse anions, which simulates disinfection by promotion of laminar flow from the interior of equipment.

In preferred embodiments, air flow for driving ozone by the main air channel 8 and vapor, oxygen and hydrogen peroxide extracted in the generation box 1 by the Venturi effect converge and are added into the mixing channel 4. The mixing channel 4 is coated with TiO₂, and meanwhile the UV/LED lamp 9 is disposed at an inlet of the mixing channel 4 and may illuminate the mixing channel with lamplight, so that the mixing channel generates more hydroxyl radical. Then, the generated gas of the mixing channel is mixed up in the atomization physical-chemical reactions of the generation box 2, which are illuminated by the UV/LED lamp disposed on the generation box 2, and meanwhile the tail end of the generation box 2 is coated with TiO₂.

The device of the disclosure comprises an electric or optical detecting unit configured to control a UV wavelength. A wavelength that the device may have could be, as shown in Table 1, 254 nm, 190 nm or less than 242 nm.

The device of the disclosure comprises concave parts for containing the generation box 1 and the generation box 2. In addition, the concave parts may allow these boxes to be inserted and placed in a device body and may provide connection and power for the generation boxes, thereby supplying power to the piezoelectric membranes for ultrasonic decomposition and atomization and controlling the piezoelectric membranes.

Logistically, there is always a generation of anions for generating ozone by high voltage in all corona effects, but under the control of a concentration of 100,000 pcs/cc to 10,000,000 pcs/cc, ozone is discharged through a propeller, a Venturi pipe and a mixed reaction and discharge pipeline at a concentration controlled at 0.001-0.05 ppm in an output distance of 5 cm by propelling an air flow rate, while a concentration of hydroxyl radicals is controlled at 1,000,000 pcs/cc to 20,000,000 pcs/cc.

In the device, at a position 5 cm away from the ozone generator, a volume of ozone is 0.0001 g to 0.5 g, and an evaporation capacity of H₂O₂ is controlled within a range of 0.001 g to 0.5 g, and a concentration of hydroxyl radicals is 500,000 pcs/cc to 20,000,000 pcs/cc.

In consideration of a current set standard limit (WEL) of hydrogen peroxide of 1.4 mg/m³ (1 ppm), an equivalent of the level of hydroxyl radical is (1.4 mg/m³)/(34 g/mol)=0.0000411 mol/m³.

By numerical calculation, a quantity 0.0000411 mol/m³×6.23×10²³=2.56×10¹⁹ molecules/m³ is acquired. If each hydrogen peroxide molecule generates two free radicals (1), a quantity 2.56×10¹⁹×2=5.12×10¹⁹/m³ is acquired, which is equivalent to the maximum radical yield of 5.12×10¹³/mL.

Then, a quantity (0.008 mols) × (6.023×10²³)×2=9.63×10²¹ is acquired by calculating generated free radicals according to reactions of interadonal gas and hydrogen peroxide in the disclosure, and if the device continuously works within 60 days, 1.6×10²⁰ free radicals are acquired every day in average.

If an interadonal operation area is 300 m³ large, a daily yield of free radicals is 1.6×10¹²/300 m³=5.33×10¹⁷ free radicals/day/m³, which is equivalent to a quantity 5.33×10¹¹/mL of free radicals every day.

The purification and hydroxyl radical generation device of the disclosure may transmit free radicals O°, H° and OH° and generate these free radicals by different reactions to disinfect and clean a whole large indoor space. The device of the disclosure may acquire the maximum number of free radicals and remove radon in the air to the maximum extent to sufficiently purify and disinfect air and surface and heat air, thereby creating comfortable living conditions.

It should be understood that although the disclosure has been described as above and precise structures shown in accompanying drawings, they are not intended to limit the disclosure. Various modifications and variations can be made without departing from the scope of the disclosure. The scope of the disclosure is only limited by claims.

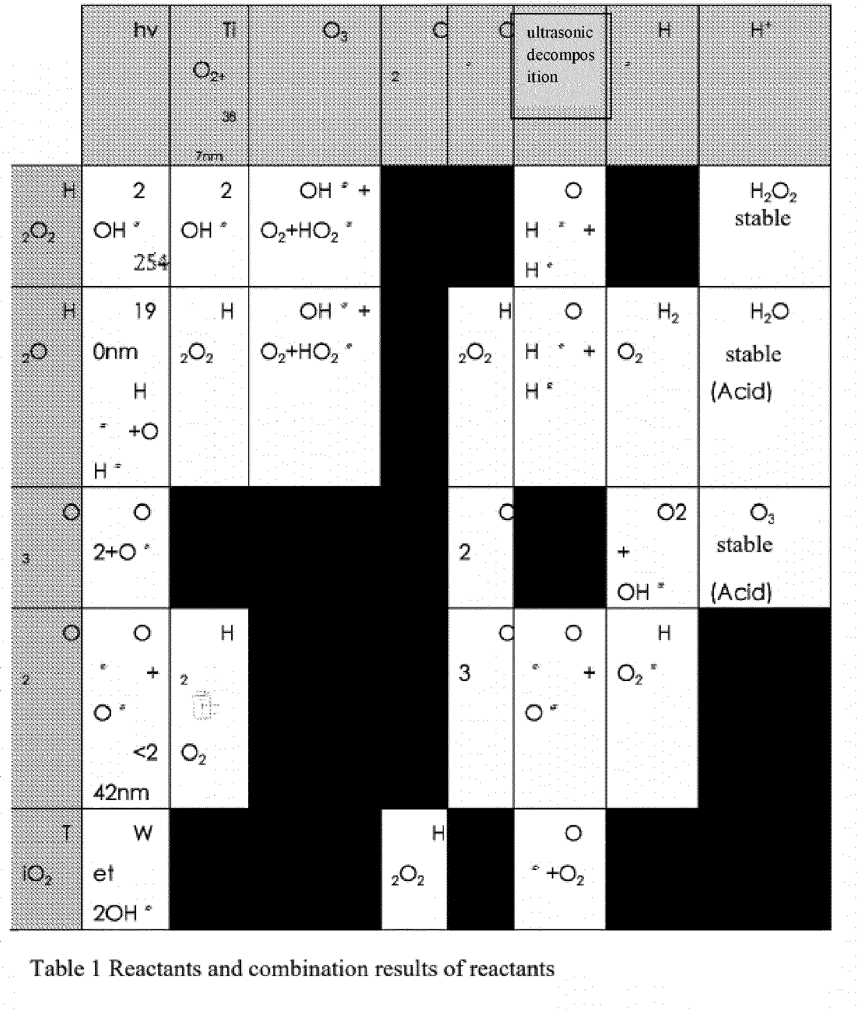

## Claims

1. A device for cleaning and sterilizing air and object surfaces, comprising:
an air control part for an air inlet that sucks air from an exterior connected to a propeller fan;
a main air channel configured to allow air to flow and generate ozone by an ozone generator and contain the ozone;
a Peltier structure communicating with the main air channel and configured to allow air to enter and condense the air;
a Venturi channel configured to bring the gas generated in a generation box 1 into a mixing area coated with TiO2 by a Venturi effect and make the gas mixed with the gas in the main air channel in the mixing area; and
a UV/LED lamp located above the main air channel and configured to illuminate the mixing area; wherein
in the mixing area, hydroxyl radicals are obtained through reactions between the UV/LED lamp and a TiO2 physical environment formed with a TiO2 coating; and
the gas in the mixing area enters a generation box 2 by the Venturi effect and is discharged from the generation box 2 into external air.

2. The device according to claim 1, wherein the generation box 1 and the generation box 2 contain hydrogen peroxide and low-electric-conductivity deionized water without carbonate or bicarbonate.

3. The device according to claim 1, comprising an electric or optical detecting device configured to control a wavelength of the UV/LED lamp so as to adjust the wavelength of the UV/LED lamp to be less than 390 nm.

4. The device according to claim 1 or 2, comprising concave parts for containing the generation box 1 and the generation box 2, wherein the concave parts may allow the generation boxes to be inserted and placed in a device body and may provide connection and power for the generation boxes.

5. The device according to claim 1 or 2, wherein a piezoelectric membrane for generating ultrasonic waves is disposed in the generation box 2 and placed on the top of the generation box 2, and meanwhile the top of the generation box 2 is coated with TiO2.

6. The device according to claim 4, wherein an ultrasonic wave generation part of a piezoelectric membrane supplies ultrasonic waves by a shell body at the upper portion of the generation box 2 for vibration for ultrasonic decomposition. An electrical part is further disposed and comprises a supply circuit and an oscillator providing an ultrasonic decomposition frequency, and the oscillator may provide an oscillation frequency consistent with a chitin frequency.

7. The device according to claim 4, wherein a piezoelectric membrane has a frequency of 15 Khz to 10 Mhz and has three effects of (i) ultrasonic decomposition, (ii) resonance with chitinous substances for weakening frameworks of mildew and yeast and eliminating aforementioned harmful substances, and (iii) atomization of liquid from the generation box 2.

8. The device according to claim 1 or 2, wherein the generation box 1 is equipped with a suction core and a piezoelectric membrane at same positions as the generation box 2 and is coated with TiO2 and illuminated by the UV/LED lamp in the same way.

9. The device according to claim 1 or 2, wherein the Peltier structure comprises a collecting unit.

10. The device according to claim 9, wherein the collecting unit is airtight porous sponge.

11. The device according to claim 10, wherein water drops in the collecting unit are placed in other places without harming human health so as to attenuate radon.

12. The device according to claim 1 or 2, wherein hot air flow may be acquired near the device and supplied out of the device by another fan, thereby raising the temperature of surrounding air of the device.

13. The device according to claim 1 or 2, wherein the generation boxes comprise piezoelectric membranes with a frequency of 15 Khz and 10 Mhz.

14. The device according to claim 13, wherein the piezoelectric membranes have a frequency resonating with an oscillation frequency of chitin exoskeleton of pillbug, thereby eliminating the pillbug.
